Europäisches Patentamt

(19) European Patent Office  (11) Publication number: **0 054 221**

Office européen des brevets  **B1**

(12) **EUROPEAN PATENT SPECIFICATION**

(45) Date of publication of patent specification: **09.04.86**  (51) Int. Cl.⁴: **A 61 J 1/00**

(21) Application number: **81110065.0**

(22) Date of filing: **02.12.81**

(54) **Multiple blood bag system made of plastic substantially free of blood extractible plasticizers.**

(30) Priority: **15.12.80 US 216098**

(43) Date of publication of application:
**23.06.82 Bulletin 82/25**

(45) Publication of the grant of the patent:
**09.04.86 Bulletin 86/15**

(84) Designated Contracting States:
**DE FR GB IT NL**

(56) References cited:
**EP-A-0 019 264
EP-A-0 026 912
BE-A- 881 623
DE-B-2 503 182
FR-A-2 439 589
GB-A-2 035 093
GB-A-2 044 220
NL-A-7 707 581
US-A-3 956 220
US-A-4 045 431
US-A-4 177 182**

**Chemical Abstracts Volume 78, Number 7,
19.02.73, page 286, abstract number, 40968**

(73) Proprietor: **MILES LABORATORIES, INC.
1127 Myrtle Street
Elkhart Indiana 46514 (US)**

(72) Inventor: **Carmen, Raleigh A.
1516, Criquet Court
Concord California 94518 (US)**

(74) Representative: **Senftl, Hannes, Dr. et al
c/o Bayer AG Konzernverwaltung RP
Patentabteilung
D-5090 Leverkusen Bayerwerk (DE)**

Courier Press, Leamington Spa, England.

# 0 054 221

**Description**

Field of the invention

This invention relates to an improvement in a multiple bag system for the collection, storage or processing of whole blood into various blood components under sterile conditions.

Prior art

Multiple blood bag systems currently available are made of polyvinyl chloride (PVC) plasticized with di-2-ethylhexyl phthalate (DEHP). Whole blood can be stored at about 5°C. for up to 21 days in bags of such a formulation without significantly reducing the quantity of surviving red cells so that the blood can be used within this period of time for transfusion.

However, there are some who believe there may be deleterious effects if DEHP, which is known to be extracted into blood, finds its way into the patient receiving blood transfusions or blood components. Consequently, efforts have been made to find other polymeric substances or formulations for blood bags which have the desirable physical properties of PVC plasticized with DEHP but which would be free of DEHP or blood extractable substances.

U.S. Patent No. 4,222,379 discloses a multiple blood bag system in which the blood storage bag (donor bag) is made of conventional PVC formulation containing DEHP plasticizer. This bag is connected to one or more transfer bags made of a polymeric substance having a different polymeric entity from that of the donor bag and contains no blood-extractable plasticizers. Thus, using this multiple blood bag system in the processing of blood into its components, red blood cells stored for the usual 21 days in the donor bag, being exposed to DEHP, maintain a sufficiently high level of survival for clinical effectiveness. It was discovered that it was the DEHP extracted or leached from the plastic by the blood which was responsible for inhibiting hemolysis of the red cells and thus allowed them to be stored for 21 days without generating significant amounts of hemoglobin. Thus the high degree of survival appeared to be dependent upon the presence of DEHP. Plasma and other blood components other than red cells such as platelets and cryoprecipitate which are in the transfer bags would not be in contact with DEHP and therefore these components at least would contain essentially no DEHP and patients receiving these products would be exposed to little risk of DEHP contamination. This patent also discloses that certain polymeric entities used in the transfer bags may have desirable characteristics, such as the capability for an increased diffusion rate of carbon dioxide through the walls of the bag which results in a decrease in the lowering of pH, a condition which favors platelet survival. Another desirable characteristic is good-low temperature strength in a transfer bag used for collecting cryoprecipitate. Certain polyethylene formulations and a blend of polyolefin with a block copolymer such as is described in U.S. Patent No. 4,140,162 are indicated in the 4,222,379 patent as having one or both of these two characteristics. Other possible transfer bag materials disclosed are polyesters, polyurethanes, poly(ethylene-vinyl acetate) copolymers, and a PVC plasticized with tri-ethylhexyl mellitate.

The system described in Patent No. 4,222,379 still has the drawback of contamination of the red cells or whole blood with DEHP.

European Patent Application Serial No. 80 105 930.4 (published April 15, 1981) is concerned with bags of PVC containing a particular non-extractable plasticizer for storage of platelets, i.e. with one of the transfer bags of the multiple blood bag system according to US—A 4,222,379. However, there is no suggestion in the prior art to make the donor bag of a plastic which is free of blood-extractable plasticizers and which has a certain minimum $CO_2$ diffusion capacity. In fact, the prior art mentioned above clearly teaches away from using such a material for donor bags.

Description of the invention

The present invention comprises a multiple blood bag system (10) comprising a donor bag (12) for storage of whole blood or red blood cells, one or more transfer bags (14, 16) for the storage of plasma, platelets and further blood components other than red blood cells, and a flexible conduit (18) joining the donor bag (12) and the transfer bag(s) (14, 16) for fluid flow communication therebetween, wherein the donor and transfer bags (12, 14, 16) are made of a flexible, translucent, heat-sterilizable, heat-sealable polymeric substance and wherein the transfer bag(s) (14, 16) exhibit a relatively high carbon dioxide transmissibility, characterized in that both the donor and transfer bags (12, 14, 16) exhibit a carbon dioxide transmissibility of at least 3000 ml/m²/day and do not comprise polymeric substances other than materials which are substantially free of blood extractable plasticizers.

Polymeric substances useful for the purposes of the invention have a $CO_2$ transmissibility of at least 3000 ml/m²/day, preferably 3000—6000 ml/m²/day ($CO_2$ transmissibility may be determined according to the method disclosed in U.S.—Patent 4,280,497). This means that the pH of the stored platelets is maintained greater than 6.0 and preferably in the range of 6.0—7.0.

In European application Serial No. 80 105 930.4, there was disclosed a bag made of polyvinyl chloride (PVC) plasticized with tri-2-ethylhexyl trimellitate (TOTM) which greatly improved survival time of blood platelets stored therein.

In U.S. Patent 4,222,379 referred to above, a PVC formulation containing tri-ethylhexyl mellitate was disclosed in which blood stored in bags of this formulation had the highest degree of hemolysis as

2

compared with blood stored in bags of different polymeric substances. It was suggested that this PVC formulation could be used in a transfer bag and the implication would automatically follow that this formulation would be unacceptable for donor bags for the storage of blood.

It was with some surprise, therefore, that when blood was stored for 21 days at 5°C. in a bag made of PVC plasticized with TOTM, it was observed that even though there was a certain amount of hemolysis occurring, the level of unhemolyzed red cells was such that the blood would provide clinically acceptable amounts of viable red cells for transfusion purposes. Thus a multiple blood bag system in which the bags were made only of PVC plasticized with TOTM would be perfectly adequate for storage of blood or red cells as well as for prolonged storage of platelets. The use of this PVC-TOTM formulation in a donor bag for storage of blood is distinctly superior to PVC bags plasticized with DEHP with respect to amounts of extractable plasticizer. With the former, only about 0.1 part per million of TOTM was detected while with the PVC-DEHP bags, 38.6±11.6 parts per million of DEHP was extracted into the blood. Thus the PVC-TOTM formulation can be considered to be substantially free of blood extractable plasticizer.

Thermoplastic polyurethane elastomers are also useful in a multiple blood bag system. For example, a polyurethan such as Texin® polyurethane manufactured by Mobay Chemical Corporation has desirable properties which render it suitable both for donor and for transfer bags. This material is much stronger than PVC and therefore bags can be fabricated from much thinner film and allow for very high carbon dioxide and oxygen transmission rates. The material is readily steam-sterilizable and is very suitable for processing blood components at cryogenic temperatures. It is free of blood extractable plasticizers such as DEHP. Thermoplastic polyurethanes are well-known in the art and various types of materials are commercially available. Their suitability for the present invention may be easily determined by means of a preliminary test as to their $CO_2$ transmissibility.

Thermoplastic polyesters, which meet the aforementioned requirements, are e.g. polyesters of 1,4-cyclohexanedicarboxylic acid and 1,4-cyclohexanedimethanol modified with 10 to 30 mole percent dimer acid, in particular polyesters having an inherent viscosity of at least 0,6 and a melting point of at least 140°C, made from

A) a dicarboxylic acid component which is

1. from 90 to 70 mole percent 1,4-cyclohexanedicarboxylic acid having a trans isomer content of at least 80 percent, and
2. from 10 to 30 mole percent dimer acid, and

B) 1,4-cyclohexanedimethanol having a trans isomer content of at least 60 percent.

These polyesters are suitable for both donor and transfer bags in a multiple blood bag system. They contain no blood extractable plasticizers such as DEHP and yet it has been found that, although some hemolysis of red cells occur when blood is stored in bags of this material for 21 days, there still remain adequate levels of unhemolyzed cells.

Thermoplastic polycarbonates free of blood-extractable plasticizer, which are blood compatible and which are sterilizable, sealable, flexible and having good gas transmissible properties, do not cause an inordinate amount of hemolysis of red cells when blood is stored for prolonged periods in bags made of this material, and, therefore, are also acceptable as the polymeric substance for a multiple blood bag system. Polycarbonates of this type are also widely available.

It is understood that the donor bag may be made of one polymeric substance and one or more interconnecting transfer bags may be made of a different polymeric substance. Thus, for example, the donor bag could be made of a polyester of the type described above and the transfer bag(s) made of a PVC-TOTM plasticized formulation as described below. A multiple blood bag system in which there are three interconnecting bags could be assembled where each of the bags was made of a different polymeric substance selected from those disclosed above.

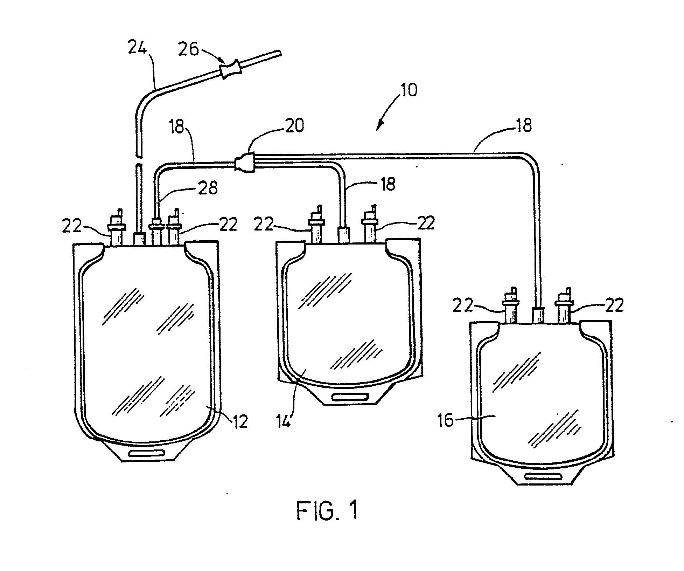

Referring to the drawings, Figure 1 is a plan view of a multiple blood bag system in accordance with this invention.

Multiple blood bag system 10 has a donor bag 12 connected to transfer bags 14 and 16 by means of flexible tubing 18 and a conventional Y connector 20. Both donor bag 12 and transfer bags 14 and 16 are equipped with hermetically sealed access ports 22 and donor bag 12 further has a blood collection tube 24 to the outer end of which is secured a donor needle assembly 26. Fluid flow through tubing 18 from donor bag 12 is controlled by conventional valving means 28, such as a ribbed spike and membrane valve.

Donor bag 12 and transfer bags 14 and 16 are made of polymeric substances which are flexible, translucent (e.g. transparent), heat-sealable, heat-sterilizable and which contain substantially no blood-extractable plasticizers. This means that the polymeric substance is either not plasticized or, if a plasticizer is present, that substantially none of the plasticizer is extracted by blood stored in bags thereof over a period of 21 days at about 5°C. Although Figure 1 shows a three bag system, the invention also is intended to include blood bag systems in which there are either two bags or four bags connected together.

A preferred embodiment

One of the preferred polymeric substances for the multiple blood bag system of this invention is polyvinyl chloride plasticized with tri-2-ethylhexyl trimellitate (TOTM). Preferably this plasticizer is present

3

to the extent of about 30 to about 50 percent in the formulation, with about 37 percent being most preferred. 3 to 5% by weight of a heat stabilizing system and other ingredients may also be present in this formulation such as minor amounts of epoxidized vegetable oils, e.g., about 3.5 percent of epoxidized soybean oil, and small quantities of metal soaps, e.g. about 0.6 percent of calcium or zinc stearate or mixtures thereof.

When blood was stored in 18 donor bags made of this preferred formulation of PVC-TOTM for 21 days at 5°C., TOTM was not detected in eight samples of blood (>0.1 ppm) and only 0.1 to about 0.15 ppm of TOTM was detected in the other ten samples. By comparison, 38.6±11.6 ppm of DEHP was detected in blood stored in PVC-DEHP bags (12 samples).

The amount of hemolysis of red cells was determined *in vitro* on blood stored for 21 days at 5°C. in bags of two commercial formulations of PVC-DEHP. The results are as follows:

| Bags of | Plasma hemoglobin, mg./100 ml |
|---|---|
| PVC-TOTM | 45.0 (Aver. of 27 units) (1) |
| PVC-DEHP (Commercial I) | 19.1 (Aver. of 10 units) (2) |
| PVC-DEHP (Commercial II) | 29.2 (Aver. of 10 units) (3) |

(1) Private communication
(2) Bailey, D.N., et al, Transfusion 15, p. 224 (1975)
(3) Private communication

Even at the highest average of 45 mg./100 ml. hemoglobin for the PVC-TOTM formulation, this is equivalent to about 0.2 percent hemolysis and would be considered to be of no clinical significance.

Of greater significance was the surprising results of *in vivo* red cell survival as determined by the [51]Cr method (Button, L. N., et al, Transfusion 5, p. 143 (1965)) on packed cells or whole blood which had been stored for 21 days at 5°C. in bags of the preferred formulation of PVC-TOTM.

| Component | No. tested | Recovery, % | Half-life, days |
|---|---|---|---|
| Whole blood | 21 | 82.6±7.2 | 30.3±5.7 |
| Packed cells 70—85% Hct (1) | 20 | 81.9±9.2 | 30.2±7.7 |
| Packed cells, >85% Hct | 9 | 82.6±12.7 | 29.2±3.8 |

(1) Hematocrit

These data comfortably meet the requirement accepted by the industry of not less than 70% post-transfusion survival. They also compare favorably to values of approximately 82% in 15 studies by Ambrus et al (Ann. N.Y. Acad. Sci. 255, p. 435 (1975)) and of approximately 80% by Gibson et al (Am. J. Clin. Path. 28, p. 569 (1957)) on red cell survival of blood stored in commercially available bags of PVC-DEHP.

The same PVC-TOTM formulation is well suited as a material for a transfer bag as well, particularly for platelet storage. As has been disclosed in the aforementioned European Patent Application Serial No. 80105930.4, viability of platelets can be maintained for considerably longer periods than presently possible when platelets are stored in bags of this PVC-TOTM formulation. This is believed to be in part the result of a diminution in the lowering of pH brought about by greater transmittability of carbon dioxide through the walls of the bag made of this formulation. Presently, storage life of platelets stored in PVC-DEHP bags is only three days or less. The bags are generally agitated gently at about room temperature and under these conditions about 300 ppm of DEHP is extracted into the platelet-plasma concentrate. Under similar conditions, using bags of PVC-TOTM instead, only about 20 ppm or less of the TOTM plasticizer is extracted into the concentrate.

Transfer bags made of the PVC-TOTM formulation are also useful in the processing of cryoprecipitate and are at least the equal to present commercial PVC-DEHP formulation bags in this respect.

Other useful embodiments

The use of other polymeric substances which contain no blood-extractable plasticizers in the formation of both donor and transfer bags is evident from the following information.

1) Oxygen and carbon dioxide transmissibility through bags made of a polyurethane (Texin® 990 AR), a polycarbonate, and a polyester (U.S. Patent No. 4,045,431), were all as good as or superior to the transmissibility of the PVC-TOTM formulation described above.

2) Plasma hemoglobin levels of blood stored for 21 days at 5°C. in bags made of these materials are as follows:

| Bag material | Plasma hemoglobin, mg./100 ml. |
|---|---|
| Polyurethane (Texin® 990 Ar) | 46.0 (Aver. of 3 units) |
| Polycarbonate | 37.0 (Aver. of 3 units) |
| Polyester | 54.0 (Aver. of 5 units) |

The above disclosure is intended for illustrative purposes only and is not intended to limit the invention which is defined in the claims which follow.

**Claims**

1. A multiple blood bag system (10) comprising a donor bag (12) for storage of whole blood or red blood cells, one or more transfer bags (14, 16) for the storage of plasma, platelets and further blood components other than red blood cells, and a flexible conduit (18) joining the donor bag (12) and the transfer bag(s) (14, 16) for fluid flow communication therebetween, wherein the donor and transfer bags (12, 14, 16) are made of a flexible, translucent, heat-sterilizable, heat-sealable polymeric substance and wherein the transfer bag(s) (14, 16) exhibit a relatively high carbon dioxide transmissibility, characterized in that both the donor and transfer bags (12, 14, 16) exhibit a carbon dioxide transmissibility of at least 3000 $ml/m^2$/day and do not comprise polymeric substances other than materials which are substantially free of blood extractable plasticizers.

2. A multiple blood bag system according to claim 1, characterized in that the polymeric substance of at least one bag is a polyvinyl chloride plasticized with tri-2-ethylhexyl trimellitate.

3. A multiple blood bag system according to claim 2, characterized in that the amount of tri-2-ethylhexyl trimellitate is 30 to 50 percent by weight.

4. A multiple blood bag system according to claim 2 or 3, characterized in that the plasticized polyvinyl chloride formulation includes about 3.5 percent of epoxidized soybean oil and about 0.6 percent of calcium or zinc stearate or combinations thereof.

5. A multiple blood bag system according to claim 1, characterized in that the polymeric substance of at least one bag is a polyurethane.

6. A multiple blood bag system according to claim 1, characterized in that the polymeric substance of at least one bag is a polyester.

7. A multiple blood bag system according to claim 6, characterized in that the polyester comprises a polymer of 1,4-cyclohexanedimethanol and 1,4-cyclohexanedicarboxylic acid.

8. A multiple blood bag system according to claim 1, characterized in that the polymeric substance of at least one bag is a polycarbonate.

9. A multiple blood bag system according to any of claims 1 to 8, characterized in that the donor and transfer bags are made of the same polymeric substance.

10. A multiple blood bag system according to any of claims 1 to 8, characterized in that the polymeric substance of donor and transfer bags is different.


**Patentansprüche**

1. Mehrfach-Blutsacksystem (10), umfassend einen Spendersack (12) zum Aufbewahren von Gesamtblut oder roten Blutzellen, einen oder mehrere Übertragungssäcke (14, 16) für die Aufbewahrung von Plasma, Plättchen und weiteren Blutkomponenten, ausgenommen rote Blutzellen, und dazwischen eine flexible Leitung (18), welche den Spendersack (12) und den oder die Übertragungssäcke (14, 16) für eine Fluidflusskommunikation verbindet, worin die Spender- und Übertragungssäcke (12, 14, 16) aus einer flexiblen, durchsichtigen, hitzesterilisierbaren, heissiegelbaren, polymeren Substanz hergestellt sind und worin der Übertragungssack oder die Übertragungssäcke (14, 16) eine verhältnismässig hohe Kohlendioxiddurchlässigkeit aufweisen, dadurch gekennzeichnet, dass sowohl der Spendersack als auch die Übertragungssäcke (12, 14, 16) eine Kohlendioxiddurchlässigkeit von wenigstens 3.000 $ml/m^2$/Tag aufweisen und keine polymeren Substanzen, ausser Materialien, die im wesentlichen frei von blutextrahierbaren Weichmachern sind, umfassen.

2. Mehrfach-Blutsacksystem gemäss Anspruch 1, dadurch gekennzeichnet, dass die polymere Substanz bei wenigstens einem Sack ein mit Tri-2-ethylhexyltrimellitat weichgemachtes Polyvinylchlorid ist.

3. Mehrfach-Blutsacksystem gemäss Anspruch 2, dadurch gekennzeichnet, dass die Menge an Tri-2-ethylhexyltrimellitat 30 bis 50 Gew.% beträgt.

4. Mehrfach-Blutsacksystem gemäss Ansprüchen 2 oder 3, dadurch gekennzeichnet, dass die Formulierung für das weichgemachte Polyvinylchlorid etwa 3,5% epoxidiertes Sojabohnenöl und etwa 0,6% Kalcium- oder Zinkstearat oder eine Kombination davon einschliesst.

5. Mehrfach-Blutsacksystem gemäss Anspruch 1, dadurch gekennzeichnet, dass die polymere Substanz bei wenigstens einem Sack ein Polyurethan ist.

6. Mehrfach-Blutsacksystem gemäss Anspruch 1, dadurch gekennzeichnet, dass die polymere Substanz bei wenigstens einem Sack ein Polyester ist.

7. Mehrfach-Blutsacksystem gemäss Anspruch 6, dadurch gekennzeichnet, dass der Polyester ein Polymer aus 1,4-Cyclohexandimethanol und 1,4-Cyclohexandicarbonsäure ist.

8. Mehrfach-Blutsacksystem gemäss Anspruch 1, dadurch gekennzeichnet, dass die polymere Substanz bei wenigstens einem Sack ein Polycarbonat ist.

9. Mehrfach-Blutsacksystem gemäss einem der Ansprüche 1 bis 8, dadurch gekennzeichnet, dass die Spender- und Übertragungssäcke aus der gleichen polymeren Substanz hergestellt sind.

10. Mehrfach-Blutsacksytem gemäss einem der Ansprüche 1 bis 8, dadurch gekennzeichnet, dass die polymeren Substanzen für den Spendersack und die Übertragungssäcke verschieden sind.

## Revendications

1. Système (10) à multiples poches de sang, comprenant une poche (12) de donneur pour conserver du sang entier ou des globules rouges du sang, une ou plusieurs poche(s) (14, 16) de transfert pour conserver le plasma, les plaquettes et d'autres constituants du sang autres que les globules rouges du sang, et un conduit flexible (18) reliant la poche (12) du donneur et la ou les poches (14, 16) de transfert pour établir une communication d'écoulement de fluide entre ces poches, la poche du donneur et les poches de transfert (12, 14, 16) étant réalisées en une substance polymère flexible, translucide, stérilisable par chauffage, scellable à chaud, et la ou les poches (14, 16) de transfert présentant une capacité relativement grande de transmission du gaz carbonique, système caractérisé en ce que la poche de donneur ainsi que les poches de transfert (12, 14, 16) présentent une capacité de transmission du gaz carbonique d'au moins 3000 ml/m$^2$/jour et ne comprennent pas de substances polymères autres que des matières essentiellement dépourvues de plastifiants extractibles par le sang.

2. Système à multiples poches de sang selon la revendication 1, caractérisé en ce que la substance polymère d'au moins une poche est un poly(chlorure de vinyle) plastifié par du trimellitate de tris(2-éthylhexyle).

3. Système à multiples poches de sang selon la revendication 2, caractérisé en ce que la quantité de trimellitate de tris(2-éthylhexyle) est de 30 à 50% en poids.

4. Système à multiples poches de sang selon la revendication 2 ou 3, caractérisé en ce que la formulation de poly(chlorure de vinyle) plastifié comprend environ 3,5% d'huile de soja époxydée et environ 0,6% de stéarate de calcium ou de zinc ou de leurs combinaisons.

5. Système à multiples poches de sang selon la revendication 1, caractérisé en ce que la substance polymère d'au moins une poche est un polyuréthanne.

6. Système à multiples poches de sang selon la revendication 1, caractérisé en ce que la substance polymère d'au moins une poche est un polyester.

7. Système à multiples poches de sang selon la revendication 6, caractérisé en ce que le polyester est ou comprend un polymère de 1,5-cyclohexanediméthanol et d'acide 1,4-cyclohexanedicarboxylique.

8. Système à multiples poches de sang selon la revendication 1, caractérisé en ce que la substance polymère d'au moins une poche est un polycarbonate.

9. Système à multiples poches de sang selon l'une quelconque des revendications 1 à 8, caractérisé en ce que les poches de donneur et de transfert sont en la même substance polymère.

10. Système à multiples poches de sang selon l'une quelconque des revendications 1 à 8, caractérisé en ce que la substance polymère est différente pour la poche du donneur et la ou les poche(s) de transfert.

FIG. 1